# EUROPEAN PATENT APPLICATION

(11) **EP 4 706 614 A1**
(43) Date of publication of application: **11.03.2026**
(21) Application number: 25192199.5
(22) Date of filing: 28.07.2025
(51) Int. Cl.: A61F 13/15, D01G 23/08, D04H 1/732

(54) **METHOD AND APPARATUS FOR PRODUCING A HYGIENIC ARTICLE**

(30) Priority: 04.09.2024 IT 202400019687
(71) Applicant: GDM S.p.A., 40133 Bologna (IT)
(72) Inventor: ZAVALLONI, Alessandro, I-40133 Bologna (IT)
(74) Representative: Porta & Consulenti Associati S.p.A.

(57) **Abstract**

The invention concerns a method for producing a hygienic article, of the type comprising a support layer and a plurality of fibres (20) arranged on said support layer. The method comprises:
- supplying a support layer strip (12) to a deposition station (120) comprising at least one deposition device (122; 222) configured to release a predetermined amount of fibres (20) per time unit;
- releasing said predetermined amount of fibres (20) from said at least one deposition device (122; 222) towards the support layer strip (12) so that a first percentage of said predetermined amount of fibres (20) falls on a deposition surface (13) of the support layer strip (12) and a remaining percentage of said predetermined amount of fibres (20) falls out of the support layer strip at the opposite sides of the support layer strip (12);
- collecting said remaining percentage of said predetermined amount of fibres (20);
- sending said remaining percentage of said predetermined amount of fibres (20) to said at least one deposition device (122; 222).

The aforesaid remaining percentage of said predetermined amount of fibres (20) varies between 5% and 60% of said predetermined amount of fibres (20), depending on the width of said support layer strip (12).

The invention also concerns an apparatus (100) suitable for carrying out the aforesaid method.

## Description

The present invention refers to a method and an apparatus for producing a hygienic article.

The hygienic article is of the type comprising a support layer and a plurality of fibres arranged on said support layer.

Non-limiting examples of hygienic articles of this type are the absorbent bed pads or the absorbent pet mats.

The absorbent pads act as a barrier between a user and the bed or other surfaces on which the user stands. Similarly, the absorbent mats act as a barrier between the pet and the litter box or other surfaces on which the pet stands. In the following description reference will be made to the non-limiting example of the absorbent bed pads, but what will be described also applies to the absorbent mats, as well as to other hygienic articles of the aforesaid type.

The absorbent bed pads are articles of assistance for the management of the incontinence. They are designed to absorb any leakage of urine or other body secretions during sleep, keeping the bed dry and comfortable.

Typically, an absorbent bed pad has a substantially rectangular shape and comprises a lower layer (also called "backsheet"), an upper layer (also called "topsheet") and an absorbent core arranged between the lower layer and the upper layer.

The lower layer is configured to be arranged on the bed and is made of a material that is impermeable to the body fluids, for example polyethylene or bioplastic.

The upper layer is configured to be arranged in a position contiguous to the user's body and is made of a material that is permeable to the body fluids. This layer is typically made of a soft, breathable material and allows the body fluid to pass through this layer and reach the absorbent core below, keeping the user dry and reducing the risk of irritations.

The absorbent core comprises a support layer and a plurality of absorbent fibres arranged on a surface of the support layer. The absorbent core has the function of absorbing the body fluids that pass through the upper layer and retaining them without leakage even for several hours.

The support layer is generally made of paper.

The absorbent fibres can be of natural or synthetic origin. Typically, these absorbent fibres comprise cellulose and/or one or more super-absorbent polymers (SAP), for example sodium polyacrylate, typically in granular form, which may be confined inside, or within a coating of, cellulose or other similar materials.

The realization of the absorbent bed pads envisages, among other things, supplying a support layer strip and depositing the aforesaid fibres on a deposition surface of the support layer strip, over the entire width of the latter.

The fibres are produced in a appropriate mill and sent from the latter to a deposition device by appropriate supply ducts.

The deposition of the fibres takes place by falling from the deposition device. This fall is aided by an air flow which, coming from the deposition device, passes through the support layer strip.

Typically, the deposition device releases a predetermined amount of fibres per unit of time. These fibres are distributed in such a way that a part of the aforesaid predetermined amount of fibres falls out of the sides of the support layer strip, externally to the latter, and another part of the aforesaid predetermined amount of fibres is deposited on the support layer strip. Regarding the fibres that are deposited on the support layer strip, a part of them is evenly distributed at a certain width of the support layer strip and another part of them is unevenly distributed at the longitudinal edges of the support layer strip. In particular, some of the fibres deposited at the longitudinal edges of the support layer strip are only partially deposited on the support layer strip.

In the following of this description and in the subsequent claims, the set formed by the fibres that fall out of the support layer strip at the opposite sides of the support layer strip and by those that are only partially deposited on the support layer strip are referred to as "excess fibres".

The Applicant has observed that the operations described above are to date carried out in apparatuses that produce a semi-finished product of predetermined width. This semi-finished product is wound in a coil, from which it is then unwound and cut to size both longitudinally and transversely to make a plurality of hygienic articles of predetermined size. In this case, the support layer strip typically has a predetermined width that is independent of the size of the hygienic articles to be made. The size of the individual hygienic articles is in fact obtained by appropriately managing the longitudinal and transverse cutting operations.

The Applicant has thought of realizing an apparatus capable of "in-line" producing hygienic articles of the type described above, carrying out the operations described above in this apparatus.

The Applicant has observed that, since in this case the hygienic articles are produced "in-line", the support layer strip supplied to the deposition device could have also very different widths depending on the size of the hygienic articles to be produced.

Consequently, with the same amount of fibres released from the deposition device, the amount of excess fibres would vary depending on the size of the hygiene articles to be produced (and therefore on the width of the support layer strip supplied from time to time to the deposition device), this variation being able to be within a range that is very wide, as well. In particular, the Applicant has calculated that, with the same amount of fibres released from the deposition device, the amount of excess fibres could vary from a minimum of 5% of the amount of fibres released from the deposition device to a maximum of 60% of the amount of fibres released from the deposition device.

Precisely by virtue of this high variability, the Applicant has thought of collecting the excess fibres and re-circulating them by sending them directly or indirectly to the deposition device.

The Applicant has also thought that, given the wide range of variability of the excess fibres collected and recirculated, it is appropriate that these excess fibres are not reintroduced into the mill to be then directed from the latter to the deposition device, but that they are recirculated in the supply duct, and therefore between the mill and the deposition device.

The Applicant has in fact observed that typically the mill is designed to produce a predetermined maximum amount of fibres and that the introduction into the mill of a high amount of excess fibres could alter the correct functioning of the mill.

The present invention therefore concerns, in a first aspect thereof, a method for producing a hygienic article, of the type comprising a support layer and a plurality of fibres arranged on said support layer.

Preferably, a support layer strip is supplied to a deposition station.

Preferably, the deposition station comprises at least one deposition device configured to release a predetermined amount of fibres per time unit.

Preferably, said predetermined amount of fibres is released from said at least one deposition device towards the support layer strip so that a first percentage of said predetermined amount of fibres falls on a deposition surface of the support layer strip and a remaining percentage of said predetermined amount of fibres falls out of the support layer strip at the opposite sides of the support layer strip.

Preferably, said remaining percentage of said predetermined amount of fibres is collected.

Preferably, said remaining percentage of said predetermined amount of fibres is sent to said at least one deposition device.

Preferably, said remaining percentage of said predetermined amount of fibres is greater than 5% of said predetermined amount of fibres, more preferably greater than 10% of said predetermined amount of fibres, even more preferably greater than 15% of said predetermined amount of fibres, depending on the width of said support layer strip.

Preferably, said remaining percentage of said predetermined amount of fibres is less than 60% of said predetermined amount of fibres.

In preferred embodiments, said remaining percentage of said predetermined amount of fibres varies between 5% and 60% of said predetermined amount of fibres, preferably between 10% and 60% of said predetermined amount of fibres, more preferably between 15% and 60% of said predetermined amount of fibres, depending on the width of said support layer strip.

In the following, when reference will be made to any range of values comprised between a minimum value and a maximum value, the aforesaid minimum and maximum values are understood to be included in the aforesaid range, unless expressly provided otherwise.

Moreover, all of the ranges include any combination of the described maximum and minimum values and include any intermediate range, even if not expressly described in detail.

Even if not expressly indicated, any numerical value is deemed to be preceded by the term "about" to also indicate any numerical value that differs slightly from that described, for example to take into account the dimensional tolerances typical of the field of reference.

In a second aspect thereof, the present invention concerns an apparatus for producing a hygienic article, of the type comprising a support layer and a plurality of fibres arranged on said support layer.

Preferably, at least one mill configured to generate a predetermined amount of fibres per time unit is provided.

Preferably, a deposition station is provided.

Preferably, at least one supply duct connecting said at least one mill to said deposition station is provided.

Preferably, there is provided at least one movement fan connected to said at least one supply duct and configured to move said fibres in said at least one supply duct along at least one supply direction oriented towards said deposition station.

Preferably, there is provided at least one recirculation duct connecting said deposition station to said at least one supply duct downstream of said at least one mill along said at least one supply direction.

Preferably, there is provided at least one movement fan that is active in said at least one supply duct.

Preferably, said at least one recirculation duct connects said deposition station to said at least one supply duct upstream of said at least one movement fan along said at least one supply direction.

Preferably, said deposition station comprises at least one deposition device configured to release said predetermined amount of fibres towards a support layer strip movable along an advancing direction so that a first percentage of said predetermined amount of fibres falls on a deposition surface of the support layer strip and a remaining percentage of said predetermined amount of fibres falls out of the support layer strip at the opposite sides of the support layer strip.

Preferably, said deposition station comprises at least one fibre collection nozzle connected to said at least one recirculation duct.

The apparatus of the invention may carry out a method according to the first aspect of the present invention.

The present invention may have, in both aspects discussed above, at least one of the preferred features described below. These features can therefore be present individually or in combination with each other, unless expressly stated otherwise, both in the method of the first aspect of the present invention and in the apparatus of the second aspect of the present invention.

Preferably, the fibres are of cellulosic material.

More preferably, the fibres are absorbent fibres.

Preferably, after having released said predetermined amount of fibres and before collecting said remaining percentage of said predetermined amount of fibres, said remaining percentage of said predetermined amount of fibres is precisely separated from said first percentage of said predetermined amount of fibres.

Preferably, said separation takes place by at least one pair of concentrated air jets.

Preferably, each of said air jets laps a respective longitudinal edge of the support layer strip.

Preferably, before supplying said predetermined amount of fibres to said deposition station, said predetermined amount of fibres is generated by at least one mill.

The mill is supplied by cellulose sheets which are shredded to generate the fibres.

Preferably, said predetermined amount of fibres is moved along a supply duct connecting said at least one mill to said deposition station.

Preferably, there is provided at least one movement fan that is active in said supply duct.

Preferably, said predetermined amount of fibres is moved from said at least one mill along at least one supply direction oriented towards said deposition station by said at least one movement fan.

Preferably, said remaining percentage of said predetermined amount of fibres is sent to said supply duct downstream of said at least one mill along said at least one supply direction.

More preferably, said remaining percentage of said predetermined amount of fibres is sent to said supply duct upstream of said at least one movement fan along said at least one supply direction.

Preferably, a first part of said predetermined amount of fibres is moved along a first supply duct connecting a first mill to a first portion of a first deposition device by a first movement fan.

Preferably, a second part of said predetermined amount of fibres is moved along a second supply duct connecting said first mill to a second portion of said first deposition device by a second movement fan.

Preferably, the first and second portion of said first deposition device are opposite along a direction orthogonal to the advancing direction of the support layer strip.

Preferably, a first part of said remaining percentage of said predetermined amount of fibres is moved along a first recirculation duct connected to said first supply duct upstream of said first movement fan along a first supply direction.

Preferably, a second part of said remaining percentage of said predetermined amount of fibres is moved along a second recirculation duct connected to said second supply duct upstream of said second movement fan along a second supply direction.

Preferably, an initial transient is provided in which said at least one mill generates an amount of fibres greater than said predetermined amount of fibres. This compensates for the fact that during this initial transient the amount of excess fibres that are recirculated is less than that which is at full capacity.

Preferably, the amount of fibres generated by the at least one mill during the initial transient is gradually reduced in the face of the progressive increase of the recirculated excess fibres. In this way, the initial transient is reduced and the rejects of production start or restart after machine downtime are reduced.

Preferably, said at least one fibre collection nozzle is movable along a first transverse direction orthogonal to the advancing direction, so as to be able to make hygienic articles of different sizes.

Preferably, said deposition station comprises at least one pair of air blowers arranged downstream of said at least one deposition device along said advancing direction, at opposite sides with respect to said advancing direction.

Preferably, each air blower is configured to emit a concentrated air jet towards said support layer strip so as to precisely separate a respective part of said remaining percentage of said predetermined amount of fibres from said first percentage of said predetermined amount of fibres.

Preferably, each air blower is movable along a second transverse direction orthogonal to the advancing direction, so as to be able to make hygienic articles of different sizes.

Preferably, said deposition station comprises a first deposition device and a second deposition device arranged downstream of said first deposition device along an advancing direction of the support layer strip.

Preferably, each of said first deposition device and second deposition device releases a respective predetermined amount of fibres per time unit towards the support layer strip.

Preferably, a respective first percentage of said respective predetermined amount of fibres falls on the deposition surface of the support layer strip at the respective deposition device.

Preferably, a respective remaining percentage of said respective predetermined amount of fibres falls out of the support layer strip at the opposite sides of the support layer strip at the respective deposition device.

Preferably, said respective remaining percentage of said respective predetermined amount of fibres is then sent to the respective deposition device.

Since the amount of excess fibres to be recirculated can be high, the Applicant has considered it advantageous to carry out the recirculation at each of said first deposition device and second deposition device.

Preferably, a first part of the predetermined amount of fibres released from said second deposition device is moved along a third supply duct connecting a second mill to a first portion of a second deposition device by a third movement fan.

Preferably, a second part of the predetermined amount of fibres released from said second deposition device is moved along a fourth supply duct connecting said second mill to a second portion of said second deposition device by a fourth movement fan.

Preferably, the first and second portions of said second deposition device are opposite along a direction orthogonal to the advancing direction of the support layer strip.

Preferably, a first part of the remaining percentage of the predetermined amount of fibres released from said second deposition device is moved along a third recirculation duct connected to said third supply duct upstream of said third movement fan along a third supply direction.

Preferably, a second part of the remaining percentage of the predetermined amount of fibres released from said second deposition device is moved along a fourth recirculation duct connected to said fourth supply duct upstream of said fourth movement fan along a fourth supply direction.

Preferably, said deposition station comprises a conveyor belt movable along a movement direction and configured to support said support layer strip in said deposition station.

Preferably, at least one pair of supply ducts, at least one pair of movement fans, at least one pair of recirculation ducts and at least one pair of fibre collection nozzles are provided.

Preferably, a first supply duct connects a first mill to a first portion of said first deposition device.

Preferably, a first movement fan is connected to said first supply duct.

Preferably, a second supply duct connects said first mill to a second portion of said first deposition device.

Preferably, the first and second portions of said first deposition device are opposite along a direction orthogonal to the advancing direction of the support layer strip.

Preferably, a second movement fan is connected to said second supply duct.

Preferably, a first recirculation duct connects a first fibre collection nozzle to said first supply duct upstream of said first movement fan along a first supply direction.

Preferably, a second recirculation duct connects a second fibre collection nozzle to said second supply duct upstream of said second movement fan along a second supply direction.

Preferably, said deposition station comprises a first deposition device and a second deposition device arranged downstream of the first deposition device along said advancing direction.

Preferably, a respective pair of supply ducts, a respective pair of movement fans and a respective pair of recirculation ducts are provided for each of said first deposition device and said second deposition device.

Preferably, a respective pair of air blowers is further provided for each of said first deposition device and said second deposition device.

Preferably, a third supply duct connects a second mill to a first portion of said second deposition device.

Preferably, a third movement fan is connected to said third supply duct.

Preferably, a fourth supply conduit connects said second mill to a second portion of said second deposition device.

Preferably, a fourth movement fan is connected to said fourth supply duct.

Preferably, the first and second portions of said second deposition device are opposite along a direction orthogonal to the advancing direction of the support layer strip.

Preferably, a third recirculation duct connects a third fibre collection nozzle to said third supply duct upstream of said third movement fan along a third supply direction.

Preferably, a fourth recirculation duct connects a fourth fibre collection nozzle to said fourth supply duct upstream of said fourth movement fan along a fourth supply direction.

Further characteristics and advantages of the present invention will become clearer from the following detailed description of a preferred embodiment thereof, made with reference to the appended drawings and provided by way of indicative and non-limiting example, in which:
- figure 1 is a schematic side elevational view of an apparatus for producing a hygienic article in accordance with the present invention;
- figure 2 is a schematic view, in cross-section and on an enlarged scale, of a part of the apparatus of figure 1, the cross-section being taken at the plane II-II of figure 1.

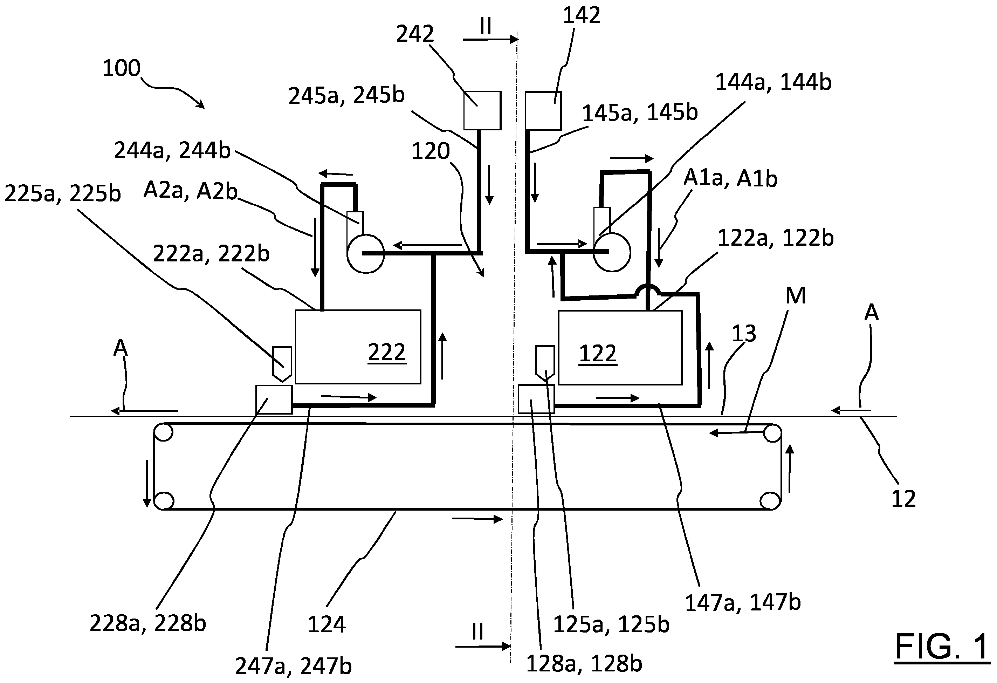

In figure 1, reference numeral 100 is used to indicate an apparatus for producing a hygienic article in accordance with the present invention.

Without for this reason losing in generality, explicit reference will be made below, as an example of a hygienic article, to an absorbent pad (not illustrated).

The absorbent pad comprises a lower layer (also called "backsheet"), an upper layer (also called "topsheet") superimposed on the lower layer and an absorbent core arranged between the lower layer and the upper layer.

In the following of the present description and in the following claims, the terms "upper", "lower", "above", "below" and the like are used referring to the position assumed by the absorbent pad when it is arranged on a bed (not illustrated), with the lower layer in contact with the bed and the upper layer arranged in such a way as to come into contact with the body of the user.

The absorbent core comprises a support layer and a plurality of fibres 20 arranged on a surface of the support layer. This surface faces the upper layer.

The support layer can be made for example of paper, while the fibres 20 can be of natural or synthetic origin. Preferably, the fibres 20 are absorbent fibres comprising cellulose and/or one or more super-absorbent polymers (SAP), for example sodium polyacrylate, typically in granular form, which may be confined inside, or within a coating of, cellulose or other similar materials.

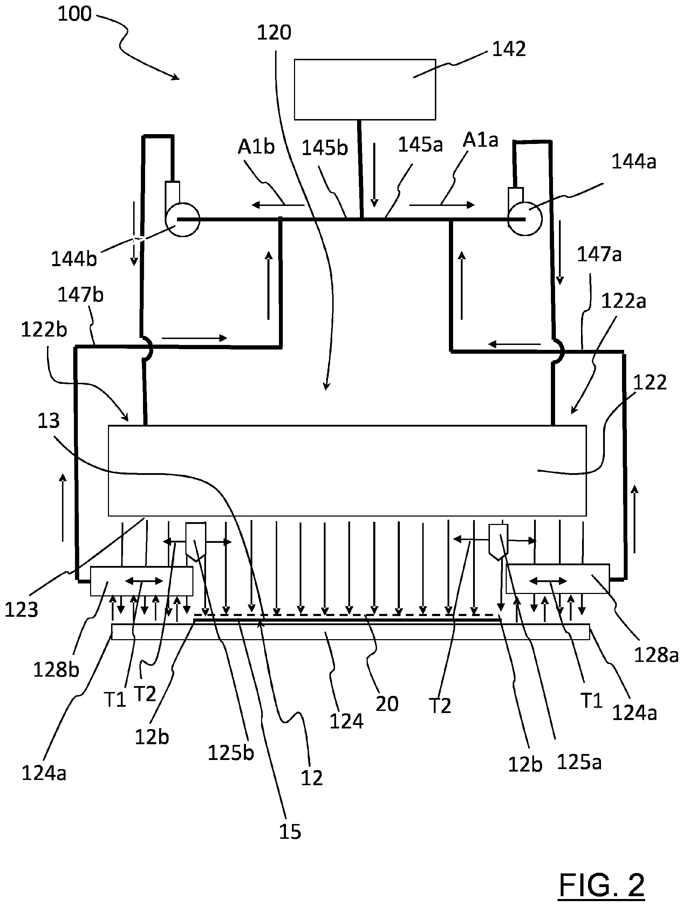

The absorbent pad is produced starting from a support layer strip 12 by means of the apparatus 100 of figures 1 and 2.

The support layer strip 12 has a deposition surface 13 destined to receive the fibres 20 and a service surface 15 opposite the deposition surface 13.

The apparatus 100 comprises a deposition station 120 in which the support layer strip 12 is supplied along an advancing direction A.

In the non-limiting example illustrated in figure 1, the deposition station 120 comprises two deposition devices 122, 222 configured to deposit the fibres 20 on the deposition surface 13. Therefore, when the support layer strip 12 passes through the deposition station 120, the deposition surface 13 faces the two deposition devices 122, 222.

The two deposition devices 122, 222 are of identical type and are arranged in sequence along the advancing direction A. The deposition device 122 positioned upstream along an advancing direction A is referred to as first deposition device 122, while the deposition device 222 positioned downstream along an advancing direction A is referred to as second deposition device 222.

The first deposition device 122 releases a predetermined amount Q1 of fibres 20 per time unit towards the support layer strip 12. A percentage of the predetermined amount Q1 of fibres 20 falls on the deposition surface 13 of the support layer strip 12 and a remaining percentage of the predetermined amount Q1 of fibres 20 falls out of the support layer strip 12 at the opposite sides of the support layer strip 12.

Similarly, the second deposition device 222 releases a predetermined amount Q2 of fibres 20 per time unit towards the support layer strip 12. A percentage of the predetermined amount Q2 of fibres 20 falls on the deposition surface 13 of the support layer strip 12 and a remaining percentage of the predetermined amount Q2 of fibres 20 falls out of the support layer strip 12 at the opposite sides of the support layer strip 12.

The predetermined amount Q1 of fibres 20 may be equal to or different from the predetermined amount Q2 of fibres 20.

The deposition station 120 also comprises a conveyor belt 124 movable along a movement direction M and configured to support the support layer strip 12 and supply it along the advancing direction A.

The support layer strip 12 rests on the conveyor belt at its service surface 15.

The conveyor belt 124 has a width greater than that of the support layer strip 12 and is arranged below the deposition devices 122, 222. Furthermore, the conveyor belt 124 is perforated. In figure 1 the support layer strip 12 is voluntarily illustrated raised with respect to the conveyor belt 124 to clearly illustrate the support layer strip 12 above the conveyor belt 124. In reality, the support layer strip 12 rests on the conveyor belt 124.

Each deposition device 122, 222 generates an air flow that facilitates the deposition of the fibres 20 on the deposition surface 13. The air flow passes through the support layer strip 12 and the conveyor belt 124.

The deposition of the fibres 20 therefore takes place by fall from each deposition device 122, 222 and by the thrust action exerted by the respective air flow.

The fibres 20 pass through an opening 123 defined in a lower surface of each deposition device 122, 222. This opening 123 preferably has a width greater than that of the support layer strip 12 and lower than or equal to that of the conveyor belt 124. As shown in figure 2, the fibres 20 that do not fall onto the support layer strip 12 and that do not deposit entirely onto the support layer strip 12 (herein referred to as "excess fibres") are sucked in at the first deposition device 122 by a pair of fibre collection nozzles 128a, 128b and at the second deposition device 222 by a pair of fibre collection nozzles 228a, 228b.

The fibre collection nozzles 128a, 128b are arranged in the first deposition device 122 above the conveyor belt 124 at opposite longitudinal sides 124a of the conveyor belt 124 and at opposite parts with respect to the latter.

Similarly, the fibre collection nozzles 228a, 228b are arranged in the second deposition device 222 above the conveyor belt 124 at opposite longitudinal sides 124a of the conveyor belt 124 and at opposite parts with respect to the latter.

The excess fibres collected by means of the fibre collection nozzles 128a, 128b are recirculated to the first deposition device 122.

Similarly, the excess fibres collected by means of the fibre collection nozzles 228a, 228b are recirculated to the second deposition device 222.

The fibre collection nozzles 128a, 128b and 228a, 228b are movable along a transverse direction T1 orthogonal to the advancing direction A so that they can be arranged in proximity of the opposite longitudinal edges 12b of the support layer strip 12 as the width of the latter varies.

A pair of air blowers 125a, 125b is arranged above the conveyor belt 124 downstream of the first deposition device 122 with reference to the advancing direction A. The air blowers 125a, 125b are positioned at opposite parts with respect to the conveyor belt 124. In particular, the air blowers 125a, 125b are positioned along a transverse direction T2 orthogonal to the movement direction M, each in proximity of a respective longitudinal side 124a of the conveyor belt 124. The transverse direction T2 is parallel to, or coincident with, the transverse direction T1, and is therefore orthogonal to the advancing direction A.

Similarly, a pair of air blowers 225a, 225b is arranged above the conveyor belt 124 downstream of the second deposition device 222 with reference to the advancing direction A. The air blowers 225a, 225b are positioned at opposite parts with respect to the conveyor belt 124. In particular, the air blowers 225a, 225b are positioned along the transverse direction T2, each in proximity of a respective longitudinal side 124a of the conveyor belt 124.

Each air blower 125a, 125b, 225a, 225b is configured to emit a concentrated air jet toward the conveyor belt 124. Each concentrated air jet laps one of the opposite longitudinal edges 12b of the support layer strip 12.

Also, but not necessarily only, due to the effect of the concentrated air jets emitted by the air blowers 125a, 125b, a percentage of the predetermined amount Q1 of fibres 20 is separated from the predetermined amount Q1 of fibres 20, sucked in by means of the fibre collection nozzles 128a, 128b and sent to the first deposition device 122.

Likewise, also, but not necessarily only, due to the effect of the concentrated air jets emitted by the air blowers 225a, 225b, a percentage of the predetermined amount Q2 of fibres 20 is separated from the predetermined amount Q2 of fibres 20, sucked in by means of the fibre collection nozzles 228a, 228b and sent to the second deposition device 222.

In accordance with the invention, the percentage of the separated predetermined amount Q1, Q2 of fibres 20 varies between 5% and 60% of the predetermined amount Q1, Q2 of fibres 20, preferably between 10% and 60% of the predetermined amount Q1, Q2 of fibres 20, even more preferably between 15% and 60% of the predetermined amount Q1, Q2 of fibres 20, depending on the width of the support layer strip 12.

For example, if each deposition device 122, 222 releases an amount of fibres 20 along a width of 900 mm and the support layer strip 12 has a width of 780 mm, the width of the two opposite zones at which the fibres 20 to be collected and recirculated fall is 120 mm, i.e. 60 mm at each longitudinal side 124a of the conveyor belt 124. In this case, the percentage of fibres 20 that is collected and recirculated at each deposition device 122, 222 is about 13.3% (calculated with the formula 120/900 x 100) of the predetermined amount Q1, Q2 of fibres 20 released from each deposition device 122, 222.

By instead supplying to the same deposition devices 122, 222 a support layer strip 12 having a width of 400 mm, the width of the two opposite zones at which the fibres 20 to be collected and recirculated fall is 500 mm, i.e. 250 mm at each longitudinal side 124a. In this case the percentage of fibres 20 that is collected and recirculated at each deposition device 122, 222 is about 55.6% (calculated with the formula 500/900 x 100) of the predetermined amount Q1, Q2 of fibres 20 released from each deposition device 122, 222.

The air blowers 125a, 125b and 225a, 225b are movable along the transverse direction T2 so that they can be arranged at the opposite longitudinal edges 12b, depending on the width of the support layer strip 12.

The apparatus 100 comprises a mill 142 configured to generate the predetermined amount Q1 of fibres 20 to be sent to the first deposition device 122 and a mill 242 configured to generate the predetermined amount Q2 of fibres 20 to be sent to the second deposition device 222.

The mill 142 is connected to a first portion 122a of the first deposition device 122 through a supply duct 145a and to a second portion 122b of the first deposition device 122 through a supply duct 145b. The second portion 122b is opposite to the first portion 122a along the transverse direction T1.

Similarly, the mill 242 is connected to a first portion 222a of the second deposition device 222 through a supply duct 245a and to a second portion 222b of the second deposition device 222 through a supply duct 245b. The second portion 222b is opposite to the first portion 222a along the transverse direction T1.

A movement fan 144a is connected to the supply duct 145a. The movement fan 144a is configured to take the fibres 20 from the mill 142 and move them in the supply duct 145a along a supply direction A1a oriented towards the deposition station 120. Another movement fan 144b is connected to the supply duct 145b. The movement fan 144b is configured to take the fibres 20 from the mill 142 and move them in the supply duct 145b along a supply direction A1b oriented towards the deposition station 120.

Similarly, a movement fan 244a is connected to the supply duct 245a. The movement fan 244a is configured to take the fibres 20 from the mill 242 and move them in the supply duct 245a along a supply direction A2a oriented towards the deposition station 120. Another movement fan 244b is connected to the supply duct 245b. The movement fan 244b is configured to take the fibres 20 from the mill 242 and move them in the supply duct 245b along a supply direction A2b oriented towards the deposition station 120.

A part of the predetermined amount Q1 of fibres 20 is moved by the movement fan 144a along the supply duct 145a. Another part of the predetermined amount Q1 of fibres 20 is moved by the movement fan 144b along the supply duct 145b.

Similarly, a part of the predetermined amount Q2 of fibres 20 is moved by the movement fan 244a along the supply duct 245a. Another part of the predetermined amount Q2 of fibres 20 is moved by the movement fan 244b along the supply duct 245b.

A pair of recirculation ducts 147a, 147b connects the deposition station 120 to the supply ducts 145a, 145b. The recirculation duct 147a connects the fibre collection nozzle 128a to the supply duct 145a downstream of the mill 142 and upstream of the movement fan 144a along the supply direction A1a, while the recirculation duct 147b connects the fibre collection nozzle 128b to the supply duct 145b downstream of the mill 142 and upstream of the movement fan 144b along the supply direction A1b.

In this way, through the recirculation ducts 147a, 147b the percentage of the predetermined amount Q1 of fibres 20 that is sucked into the fibre collection nozzles 128a, 128b, is recirculated to the first deposition device 122. In particular, a part of the aforesaid percentage is moved along the recirculation duct 147a connected to the supply duct 145a and therefore to the first portion 122a of the first deposition device 122, while another part of the aforesaid percentage is moved along the recirculation duct 147b connected to the supply duct 145b and therefore to the second portion 122b of the first deposition device 122.

Similarly, a pair of recirculation ducts 247a, 247b connects the deposition station 120 to the supply ducts 245a, 245b. The recirculation duct 247a connects the fibre collection nozzle 228a to the supply duct 245a downstream of the mill 242 and upstream of the movement fan 244a along the supply direction A2a, while the recirculation duct 247b connects the fibre collection nozzle 228b to the supply duct 245b downstream of the mill 242 and upstream of the movement fan 244b along the supply direction A2b.

In this way, through the recirculation ducts 247a, 247b the percentage of the predetermined amount Q2 of fibres 20, which is sucked into the fibre collection nozzles 228a, 228b, is recirculated to the second deposition device 222. In particular, a part of the aforesaid percentage is moved along the recirculation duct 247a connected to the supply duct 245a and therefore to the first portion 222a of the second deposition device 222, while another part of the aforesaid percentage is moved along the recirculation duct 247b connected to the supply duct 245b and therefore to the second portion 222b of the second deposition device 222.

Therefore, as illustrated in figures 1 and 2, for the first deposition device 122 there are provided a pair of supply ducts 145a, 145b, a pair of movement fans 144a, 144b, a pair of air blowers 125a, 125b and a pair of recirculation ducts 147a, 147b. Similarly, for the second deposition device 222 there are provided a pair of supply ducts 245a, 245b, a pair of movement fans 244a, 244b, a pair of air blowers 225a, 225b and a pair of recirculation ducts 247a, 247b.

In some embodiments it is provided that in an initial transient the mill 142 generates an amount of fibres greater than the predetermined amount Q1 of fibres 20 and the mill 242 generates an amount of fibres greater than the predetermined amount Q2 of fibres 20.

The apparatus 100 further comprises, downstream of the deposition station 120 along an advancing direction A of the support layer strip 12, an application station (not illustrated because of conventional type) configured to apply the lower layer strip and the upper layer strip respectively below and above the support layer strip 12.

Obviously, a person skilled in the art, in order to satisfy specific and contingent needs, can make numerous modifications and variations to the invention described above while remaining within the scope of protection defined by the following claims.

## Claims

1. Method for producing a hygienic article, of the type comprising a support layer and a plurality of fibres (20) arranged on said support layer, said method comprising:
- supplying a support layer strip (12) to a deposition station (120) comprising at least one deposition device (122; 222) configured to release a predetermined amount of fibres (20) per time unit;
- releasing said predetermined amount of fibres (20) from said at least one deposition device (122; 222) towards the support layer strip (12) so that a first percentage of said predetermined amount of fibres (20) falls on a deposition surface (13) of the support layer strip (12) and a remaining percentage of said predetermined amount of fibres (20) falls out of the support layer strip (12) at the opposite sides of the support layer strip (12);
- collecting said remaining percentage of said predetermined amount of fibres (20);
- sending said remaining percentage of said predetermined amount of fibres (20) to said at least one deposition device (122; 222);
wherein said remaining percentage of said predetermined amount of fibres (20) varies between 5% and 60% of said predetermined amount of fibres (20) depending on the width of said support layer strip (12).

2. Method according to claim 1, wherein said remaining percentage of said predetermined amount of fibres (20) varies between 10% and 60% of said predetermined amount of fibres (20) depending on the width of said support layer strip (12).

3. Method according to claim 1 or 2, comprising, after having released said predetermined amount of fibres (20) and before collecting said remaining percentage of said predetermined amount of fibres (20):
- precisely separating said remaining percentage of said predetermined amount of fibres (20) from said first percentage of said predetermined amount of fibres (20) by at least one pair of concentrated air jets.

4. Method according to any one of the previous claims, comprising, before supplying said predetermined amount of fibres (20) to said deposition station (120):
- generating said predetermined amount of fibres (20) by at least one mill (142; 242);
- moving said predetermined amount of fibres (20) from said at least one mill (142; 242) along at least one supply direction (A1a, A1b; A2a, A2b) oriented towards said deposition station (120) by at least one movement fan (144a, 144b; 244a, 244b), wherein said remaining percentage of said predetermined amount of fibres (20) is sent downstream of said at least one mill (142; 242) and upstream of said at least one movement fan (144a, 144b; 244a, 244b) along said at least one supply direction (Ala, A1b; A2a, A2b).

5. Method according to claim 4, wherein moving said predetermined amount of fibres (20) from said at least one mill (142; 242) comprises:
- moving a first part of said predetermined amount of fibres (20) along a first supply duct (145a) connecting a first mill (142) to a first portion (122a) of a first deposition device (122) by a first movement fan (144a);
- moving a second part of said predetermined amount of fibres (20) along a second supply duct (145b) connecting said first mill (142) to a second portion (122b) of said first deposition device (122) by a second movement fan (144b);
and wherein sending said remaining percentage of said predetermined amount of fibres (20) to said at least one deposition device (122; 222) comprises:
- moving a first part of said remaining percentage of said predetermined amount of fibres (20) along a first recirculation duct (147a) connected to said first supply duct (145a) upstream of said first movement fan (144a) along a first supply direction (A1a);
- moving a second part of said remaining percentage of said predetermined amount of fibres (20) along a second recirculation duct (147b) connected to said second supply duct (145b) upstream of said second movement fan (144b) along a second supply direction (A1b).

6. Method according to claim 4 or 5, comprising an initial transient in which said at least one mill (142; 242) generates an amount of fibres (20) greater than said predetermined amount of fibres (20).

7. Method according to any one of the previous claims, wherein said deposition station (120) comprises a first deposition device (122) and a second deposition device (222) arranged downstream of the first deposition device (122) along an advancing direction (A) of the support layer strip (12), wherein each of said first deposition device (122) and said second deposition device (222) releases a respective predetermined amount of fibres (20) per time unit towards the support layer strip (12) such that a respective first percentage of said respective predetermined amount of fibres (20) falls on the deposition surface (13) of the support layer strip (12) at the respective deposition device (122; 222) and a respective remaining percentage of said respective predetermined amount of fibres (20) falls out of the support layer strip (12) at the opposite sides of the support layer strip (12) at the respective deposition device (122; 222), wherein said respective remaining percentage of said respective predetermined amount of fibres (20) is then sent to the respective deposition device (122; 222).

8. Method according to claim 7 when depending on claim 5, wherein moving said predetermined amount of fibres (20) from said at least one mill (142; 242) comprises:
- moving a first part of the predetermined amount of fibres (20) released from said second deposition device (222) along a third supply duct (245a) connecting a second mill (242) to a first portion (222a) of said second deposition device (222) by a third movement fan (244a);
- moving a second part of the predetermined amount of fibres (20) released from said second deposition device (222) along a fourth supply duct (245b) connecting said second mill (242) to a second portion (222b) of said second deposition device (222) by a fourth movement fan (244b);
and wherein sending said remaining percentage of said predetermined amount of fibres (20) to said at least one deposition device (122; 222) comprises:
- moving a first part of the remaining percentage of the predetermined amount of fibres (20) released by said second deposition device (222) along a third recirculation duct (247a) connected to said third supply duct (245a) upstream of said third movement fan (244a) along a third supply direction (A2a);
- moving a second part of the remaining percentage of the predetermined amount of fibres (20) released by said second deposition device (222) along a fourth recirculation duct (247b) connected to said fourth supply duct (245b) upstream of said fourth movement fan (244b) along a fourth supply direction (A2b).

9. Apparatus (100) for producing a hygienic article, of the type comprising a support layer and a plurality of fibres (20) arranged on said support layer, said apparatus comprising:
- at least one mill (142; 242) configured to generate a predetermined amount of fibres (20) per time unit;
- a deposition station (120);
- at least one supply duct (145a, 145b; 245a, 245b) connecting said at least one mill (142; 242) to said deposition station (120);
- at least one movement fan (144a, 144b; 244a, 244b) connected to said at least one supply duct (145a, 145b; 245a, 245b) and configured to move said fibres (20) in said at least one supply duct (145a, 145b; 245a, 245b) along at least one supply direction (Ala, A1b; A2a, A2b) oriented towards said deposition station (120);
- at least one recirculation duct (147a, 147b; 247a, 247b) connecting said deposition station (120) to said at least one supply duct (145a, 145b; 245a, 245b) downstream of said at least one mill (142, 242) and upstream of said at least one movement fan (144a, 144b; 244a, 244b) along said at least one supply direction (Ala, A1b; A2a, A2b);
wherein said deposition station (120) comprises:
- at least one deposition device (122; 222) configured to release said predetermined amount of fibres (20) towards a support layer strip (12) movable along an advancing direction (A) so that a first percentage of said predetermined amount of fibres (20) falls on a deposition surface (13) of the support layer strip (12) and a remaining percentage of said predetermined amount of fibres (20) falls out of the support layer strip (12) at the opposite sides of the support layer strip (12);
- at least one fibre collecting nozzle (128a, 128b; 228a, 228b) connected to said at least one recirculation duct (147a, 147b; 247a, 247b).

10. Apparatus according to claim 9, wherein said at least one fibre collection nozzle (128a, 128b; 228a, 228b) is movable along a first transverse direction (T1) orthogonal to the advancing direction (A).

11. Apparatus according to claim 9 or 10, wherein said deposition station (120) further comprises at least one pair of air blowers (125a, 125b; 225a, 225b) arranged downstream of said at least one deposition device (122; 222) along said advancing direction (A), at opposite sides with respect to said advancing direction (A), each air blower (125a, 125b; 225a, 225b) being configured to emit a concentrated air jet towards said support layer strip (12) so as to precisely separate a respective part of said remaining percentage of said predetermined amount of fibres (20) from said first percentage of said predetermined amount of fibres (20), wherein each air blower (125a, 125b; 225a, 225b) is movable along a second transverse direction (T2) orthogonal to the advancing direction (A).

12. Apparatus according to any one of claims 9 to 11, wherein said deposition station (120) comprises a conveyor belt (124) movable along a movement direction (M) and configured to support said support layer strip (12) in said deposition station (120).

13. Apparatus according to any one of claims 9 to 12, comprising at least one pair of supply ducts (145a; 145b), at least one pair of movement fans (144a, 144b), at least one pair of recirculation ducts (147a; 147b) and at least one pair of fibre collection nozzles (128a; 128b), wherein:
- a first supply duct (145a) connects a first mill (142) to a first portion (122a) of a first deposition device (122);
- a first movement fan (144a) is connected to said first supply duct (145a);
- a second supply duct (145b) connects said first mill (142) to a second portion (122b) of said first deposition device (122);
- a second movement fan (144b) is connected to said second supply duct (145b);
- a first recirculation duct (147a) connects a first fibre collection nozzle (128a) to said first supply duct (145a) upstream of said first movement fan (144a) along a first supply direction (A1a);
- a second recirculation duct (147b) connects a second fibre collection nozzle (128b) to said second supply duct (145b) upstream of said second movement fan (144b) along a second supply direction (A1b).

14. Apparatus according to any one of claims 9 to 13, wherein said deposition station (120) comprises a first deposition device (122) and a second deposition device (222) arranged downstream of the first deposition device (122) along said advancing direction (A), wherein a respective pair of supply ducts (145a, 145b; 245a, 245b), a respective pair of movement fans (144a, 144b; 244a, 244b) and a respective pair of recirculation ducts (147a, 147b; 247a, 247b) are provided for each of said first deposition device (122) and said second deposition device (222).

15. Apparatus according to claim 14 when depending on claim 13, wherein:
- a third supply duct (245a) connects a second mill (242) to a first portion (222a) of said second deposition device (222);
- a third movement fan (244a) is connected to said third supply duct (245a);
- a fourth supply duct (245b) connects said second mill (242) to a second portion (222b) of said second deposition device (222);
- a fourth movement fan (244b) is connected to said fourth supply duct (245b);
- a third recirculation duct (247a) connects a third fibre collection nozzle (228a) to said third supply duct (245a) upstream of said third movement fan (244a) along a third supply direction (A2a);
- a fourth recirculation duct (247b) connects a fourth fibre collection nozzle (228b) to said fourth supply duct (245b) upstream of said fourth movement fan (244b) along a fourth supply direction (A2b).
